# EUROPEAN PATENT SPECIFICATION

(11) **EP 4 166 548 B1**
(45) Date of publication and mention of the grant of the patent: **28.10.2026**
(21) Application number: 22202153.7
(22) Date of filing: 25.07.2013
(51) Int. Cl.: C07D 309/10, A61P 3/10, A61K 31/351

(54) **CRYSTALLINE MONOHYDRATE COMPLEX OF 1-CYANO-2-(4-CYCLOPROPYL-BENZYL)-4-(BETA-D-GLUCOPYRANOS-1-YL)-BENZENE AND L-PROLINE IN CRYSTAL WATER (1:1;1)**
KRISTALLINER MONOHYDRAT KOMPLEX VON 1-CYANO-2-(4-CYCLOPROPYL-BENZYL)-4-(S-D-GLUCOPYRANOS-1-YL)-BENZOL UND L-PROLINE IN KRYSTALWASSER (1:1:1)
COMPLEXE MONOHYDRATE CRISTALLIN DE 1-CYANO-2-(4-CYCLOPROPYL-BENZYL)-4-(SS-D-GLUCOPYRANOS-1-YL)-BENZÈNE ET DE L-PROLINE DANS L'EAU CRISTALLINE (1:1:1)

(30) Priority: 26.07.2012 EP 12177944
(43) Date of publication of application: 19.04.2023
(62) Document number(s) of the earlier application(s) in accordance with Art. 76 EPC: 18155079.9 / 3 351 539; 13740309.3 / 2 877 460
(73) Proprietor: Boehringer Ingelheim Vetmedica GmbH, 55216 Ingelheim am Rhein (DE)
(72) Inventor: ECKHARDT, Matthias, 55216 Ingelheim am Rhein (DE); BUTZ, Tanja, 55216 Ingelheim am Rhein (DE); HIMMELSBACH, Frank, 55216 Ingelheim am Rhein (DE); MARTIN, Hans-Juergen, 55216 Ingelheim am Rhein (DE)
(74) Representative: Boehringer Global Patents

(56) References cited:
- WO-A1-2007/093610
- WO-A1-2007/128749

## Description

The invention relates to a crystalline complex of 1-cyano-2-(4-cyclopropyl-benzyl)-4-(ß-D-glucopyranos-1-yl)-benzene, to methods for the preparation thereof, as well as to the use thereof for preparing medicaments.

### Background of the invention

The compound 1-cyano-2-(4-cyclopropyl-benzyl)-4-(ß-D-glucopyranos-1-yl)-benzene (in the following referred to it as "compound A") is described in WO 2007/093610 and WO 2007/128749 and has the chemical structure according to formula A

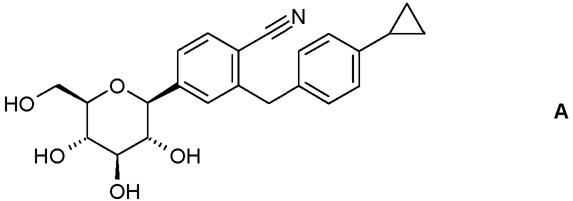

The compounds described therein have a valuable inhibitory effect on the sodium-dependent glucose cotransporter SGLT, particularly SGLT2. The method of manufacture of the compound A as described therein does not yield a crystalline form.

A certain pharmaceutical activity is of course the basic prerequisite to be fulfilled by a pharmaceutically active agent before same is approved as a medicament on the market. However, there are a variety of additional requirements a pharmaceutically active agent has to comply with. These requirements are based on various parameters which are connected with the nature of the active substance itself. Without being restrictive, examples of these parameters are the stability of the active agent under various environmental conditions, its stability during production of the pharmaceutical formulation and the stability of the active agent in the final medicament compositions. The pharmaceutically active substance used for preparing the pharmaceutical compositions should be as pure as possible and its stability in long-term storage must be guaranteed under various environmental conditions. This is essential to prevent the use of pharmaceutical compositions which contain, in addition to the actual active substance, breakdown products thereof, for example. In such cases the content of active substance in the medicament might be less than that specified.

Uniform distribution of the medicament in the formulation is a critical factor, particularly when the medicament has to be given in low doses. To ensure uniform distribution, the particle size of the active substance can be reduced to a suitable level, e.g. by grinding. Since breakdown of the pharmaceutically active substance as a side effect of the grinding (or micronising) has to be avoided as far as possible, in spite of the hard conditions required during the process, it is essential that the active substance should be highly stable throughout the grinding process. Only if the active substance is sufficiently stable during the grinding process it is possible to produce a homogeneous pharmaceutical formulation which always contains the specified amount of active substance in a reproducible manner.

Another problem which may arise in the grinding process for preparing the desired pharmaceutical formulation is the input of energy caused by this process and the stress on the surface of the crystals. This may in certain circumstances lead to polymorphous changes, to amorphization or to a change in the crystal lattice. Since the pharmaceutical quality of a pharmaceutical formulation requires that the active substance should always have the same crystalline morphology, the stability and properties of the crystalline active substance are subject to stringent requirements from this point of view as well.

The stability of a pharmaceutically active substance is also important in pharmaceutical compositions for determining the shelf life of the particular medicament; the shelf life is the length of time during which the medicament can be administered without any risk. High stability of a medicament in the abovementioned pharmaceutical compositions under various storage conditions is therefore an additional advantage for both the patient and the manufacturer.

The absorption of moisture reduces the content of pharmaceutically active substance as a result of the increased weight caused by the uptake of water. Pharmaceutical compositions with a tendency to absorb moisture have to be protected from moisture during storage, e.g. by the addition of suitable drying agents or by storing the drug in an environment where it is protected from moisture. Preferably, therefore, a pharmaceutically active substance should be at best slightly hygroscopic.

Furthermore, the availability of a well-defined crystalline form allows the purification of the drug substance by recrystallization.

Apart from the requirements indicated above, it should be generally borne in mind that any change to the solid state of a pharmaceutical composition which is capable of improving its physical and chemical stability gives a significant advantage over less stable forms of the same medicament.

The aim of the invention is thus to provide a new, stable crystalline form of the compound A which meets important requirements imposed on pharmaceutically active substances as those mentioned above.

### Object of the invention

The invention is defined by the claims and any other aspects, considerations, configurations or embodiments set forth herein not falling within the scope of the claims are for information only.

That is, the invention relates to the following aspects:
(a) A mixture of crystalline forms comprising at least one crystalline complex in admixture with another crystalline form or forms of 1-cyano-2-(4-cyclopropyl-benzyl)-4-(ß-D-glucopyranos-1-yl)-benzene, wherein the crystalline complex is a crystalline complex between 1-cyano-2-(4-cyclopropyl-benzyl)-4-(ß-D-glucopyranos-1-yl)-benzene and one or more natural amino acids:
(i) characterized in that the natural amino acid is L-proline,
(ii) characterized in that the crystalline complex is a complex (1:1) between 1-cyano-2-(4-cyclopropyl-benzyl)-4-(ß-D-glucopyranos-1-yl)-benzene and L-proline,
(iii) characterized in that the crystalline complex is a crystalline hydrate, and
(iv) characterized in that the molar ratio of the crystalline complex of 1-cyano-2-(4-cyclopropyl-benzyl)-4-(ß-D-glucopyranos-1-yl)-benzene and L-proline with crystal water is 1:1.

(b) The mixture of crystalline forms according to (a), wherein the crystalline complex is characterized by an X-ray powder diffraction pattern that comprises peaks at 20.28, 21.14 and 21.64 degrees 2Θ (±0.1 degrees 2Θ), wherein said X-ray powder diffraction pattern is made using CuK_{α1} radiation.
(c) The mixture of crystalline forms according to any one of (a) to (b), wherein the X-ray powder diffraction pattern of the crystalline complex further comprises peaks at 4.99 and 23.23 degrees 2Θ (±0.1 degrees 2Θ), wherein said X-ray powder diffraction pattern is made using CuK_{α1} radiation.
(d) The mixture of crystalline forms according to any one of (a) to (c), wherein the X-ray powder diffraction pattern of the crystalline complex further comprises peaks at 17.61, 17.77 and 27.66 degrees 2Θ (±0.1 degrees 2Θ), wherein said X-ray powder diffraction pattern is made using CuK_{α1} radiation.
(e) The mixture of crystalline forms according to any one of (a) to (d), wherein the X-ray powder diffraction pattern of the crystalline complex comprises peaks at 4.99, 15.12, 17.61, 17.77, 18.17, 20.28, 21.14, 21.64, 23.23 and 27.66 degrees 2Θ (±0.1 degrees 2Θ), wherein said X-ray powder diffraction pattern is made using CuK_{α1} radiation.
(f) The mixture of crystalline forms according to any one of (a) to (e), wherein the crystalline complex is characterised by an X-ray powder diffraction pattern, made using CuK_{α1} radiation, which comprises peaks at degrees 2Θ (±0.1 degrees 2Θ) as follows:

| **2** Θ **[°]** | **d-value [Å]** | **Intensity I/I₀ [%]** |
|---|---|---|
| 4.99 | 17.68 | 39 |
| 7.01 | 12.61 | 6 |
| 8.25 | 10.70 | 11 |
| 9.95 | 8.88 | 12 |
| 13.15 | 6.73 | 30 |
| 13.33 | 6.64 | 10 |
| 14.08 | 6.28 | 4 |
| 15.12 | 5.85 | 32 |
| 16.40 | 5.40 | 12 |
| 16.49 | 5.37 | 13 |
| 17.11 | 5.18 | 6 |
| 17.61 | 5.03 | 32 |
| 17.77 | 4.99 | 35 |
| 18.17 | 4.88 | 32 |
| 18.32 | 4.84 | 28 |
| 18.72 | 4.74 | 8 |
| 19.16 | 4.63 | 30 |
| 19.96 | 4.45 | 26 |
| 20.28 | 4.37 | 56 |
| 20.60 | 4.31 | 7 |
| 21.14 | 4.20 | 84 |
| 21.64 | 4.10 | 100 |
| 22.33 | 3.98 | 15 |
| 23.23 | 3.83 | 41 |
| 24.06 | 3.70 | 4 |
| 24.51 | 3.63 | 15 |
| 24.93 | 3.57 | 26 |
| 25.89 | 3.44 | 23 |
| 26.21 | 3.40 | 11 |
| 26.84 | 3.32 | 8 |
| 27.66 | 3.22 | 38 |
| 27.96 | 3.19 | 9 |
| 28.26 | 3.16 | 5 |
| 28.44 | 3.14 | 6 |
| 28.75 | 3.10 | 6 |
| 29.18 | 3.06 | 19 |

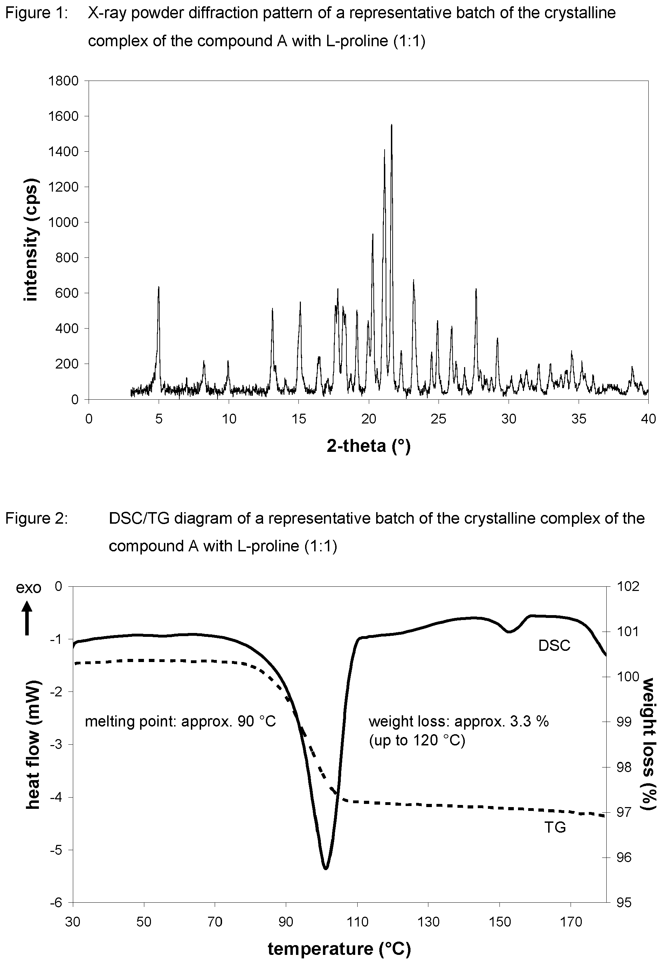

(g) The mixture of crystalline forms according to any one of (a) to (f), wherein said crystalline complex is characterised by an X-ray powder diffraction pattern, made using CuK_{α1} radiation, which comprises peaks at degrees 2Θ (±0.1 degrees 2Θ) as shown in Figure 1.
(h) The mixture of crystalline forms according to any one of (a) to (g) comprising at least 50%-weight, preferably at least 90%-weight, more preferably at least 95%-weight of the crystalline complex as defined in any one of (a) to (g).
(i) Use of the mixture of crystalline forms according to any one of (a) to (h) for the preparation of a pharmaceutical composition.
(j) The mixture of crystalline forms according to any one of (a) to (h) for use in the preparation of a pharmaceutical composition, preferably for use in the preparation of a pharmaceutical composition for the treatment or prevention of diseases or conditions which can be influenced by inhibiting the sodium-dependent glucose cotransporter SGLT, more preferably the sodium-dependent glucose cotransporter SGLT2.
(k) The mixture of crystalline forms according to any one of (a) to (h) for use as a medicament.
(l) The mixture of crystalline forms according to any one of (a) to (h) for use in the treatment or prevention of diseases or conditions which can be influenced by inhibiting the sodium-dependent glucose cotransporter SGLT, more preferably the sodium-dependent glucose cotransporter SGLT2.
(m) Use of a crystalline complex between 1-cyano-2-(4-cyclopropyl-benzyl)-4-(ß-D-glucopyranos-1-yl)-benzene and one or more natural amino acids for the preparation of a pharmaceutical composition,
(i) characterized in that the natural amino acid is L-proline, and
(ii) characterized in that the crystalline complex is a complex (1:1) between 1-cyano-2-(4-cyclopropyl-benzyl)-4-(ß-D-glucopyranos-1-yl)-benzene and L-proline, and
(iii) characterized in that the crystalline complex is a crystalline hydrate, and
(iv) characterized in that the molar ratio of the crystalline complex of 1-cyano-2-(4-cyclopropyl-benzyl)-4-(ß-D-glucopyranos-1-yl)-benzene and L-proline with crystal water is 1:1.

(n) The crystalline complex according to (m) for use in the preparation of a pharmaceutical composition, preferably for use in the preparation of a pharmaceutical composition for the treatment or prevention of diseases or conditions which can be influenced by inhibiting the sodium-dependent glucose cotransporter SGLT, more preferably the sodium-dependent glucose cotransporter SGLT2.

In the light of the pharmaceutical efficacy of the compound A and the advantageous physical chemical properties of the crystalline complex an aspect of the present invention relates to a pharmaceutical composition or medicament comprising the crystalline complex or a mixture of corresponding crystalline forms as defined hereinbefore and hereinafter.

A further aspect of the present invention relates to the crystalline complex and a mixture of corresponding crystalline forms for use as a medicament.

In a further aspect the present invention relates to a use of the crystalline complex or a mixture of corresponding crystalline forms for preparing a pharmaceutical composition which is suitable for the treatment or prevention of diseases or conditions which can be influenced by inhibiting sodium-dependent glucose cotransporter SGLT, preferably SGLT2.

In a further aspect the present invention relates to a use of the crystalline complex or a mixture of corresponding crystalline forms for preparing a pharmaceutical composition for inhibiting the sodium-dependent glucose cotransporter SGLT2.

### Brief Description of the Figures

The Figure 1 shows an X-ray powder diffractogram of the crystalline complex between the compound A and L-proline.
The Figure 2 shows the determination of the melting point via DSC and of the weight loss via TG of the crystalline complex between the compound A and L-proline.

### Detailed description of the invention

Surprisingly, it has been found that there exists a crystalline complex between a natural amino acid and the compound A. Such a complex fulfills important requirements mentioned hereinbefore. Accordingly the present invention relates to a crystalline complex between the compound A and one natural amino acid, characterized in that the natural amino acid is L-proline and characterized in that the crystalline complex is a complex (1:1) between 1-cyano-2-(4-cyclopropyl-benzyl)-4-(ß-D-glucopyranos-1-yl)-benzene and L-proline and characterized in that the crystalline complex is a crystalline hydrate and characterized in that the molar ratio of the crystalline complex of 1-cyano-2-(4-cyclopropyl-benzyl)-4-(ß-D-glucopyranos-1-yl)-benzene and L-proline with crystal water is 1:1; as well as a mixture of corresponding crystalline forms.

According to the invention the crystalline complex is characterized in that the natural amino acid is (L)-proline.

The crystalline complex according to this invention is a complex (1 : 1) between the compound A and L-proline.

The 1 : 1 complex of the compound A with L-proline, is a hydrate.

The molar ratio of the crystalline complex and water is 1 : 1.

The crystalline complex of the compound A with L-proline and water, may be identified and distinguished from other crystalline forms by means of their characteristic X-ray powder diffraction (XRPD) patterns.

Said crystalline complex is preferably characterised by an X-ray powder diffraction pattern that comprises peaks at 20.28, 21.14 and 21.64 degrees 2Θ (±0.1 degrees 2Θ), wherein said X-ray powder diffraction pattern is made using CuK_{α1} radiation.

In particular said X-ray powder diffraction pattern comprises peaks at 4.99, 20.28, 21.14, 21.64 and 23.23 degrees 2Θ (±0.1 degrees 2Θ), wherein said X-ray powder diffraction pattern is made using CuK_{α1} radiation.

More specifically said X-ray powder diffraction pattern comprises peaks at 4.99, 17.61, 17.77, 20.28, 21.14, 21.64, 23.23 and 27.66 degrees 2Θ (±0.1 degrees 2Θ), wherein said X-ray powder diffraction pattern is made using CuK_{α1} radiation.

Even more specifically said X-ray powder diffraction pattern comprises peaks at 4.99, 15.12, 17.61, 17.77, 18.17, 20.28, 21.14, 21.64, 23.23 and 27.66 degrees 2Θ (±0.1 degrees 2Θ), wherein said X-ray powder diffraction pattern is made using CuK_{α1} radiation.

Even more specifically, the crystalline complex of the compound A and L-proline is characterised by an X-ray powder diffraction pattern, made using CuK_{α1} radiation, which comprises peaks at degrees 2Θ (±0.1 degrees 2Θ) as contained in Table 1.

**Table 1: X-ray powder diffraction pattern of the crystalline complex of the compound A and L-proline (only peaks up to 30° in 2 Θ are listed):**

| **2** Θ **[°]** | **d-value [Å]** | **Intensity I/I₀ [%]** |
|---|---|---|
| 4.99 | 17.68 | 39 |
| 7.01 | 12.61 | 6 |
| 8.25 | 10.70 | 11 |
| 9.95 | 8.88 | 12 |
| 13.15 | 6.73 | 30 |
| 13.33 | 6.64 | 10 |
| 14.08 | 6.28 | 4 |
| 15.12 | 5.85 | 32 |
| 16.40 | 5.40 | 12 |
| 16.49 | 5.37 | 13 |
| 17.11 | 5.18 | 6 |
| 17.61 | 5.03 | 32 |
| 17.77 | 4.99 | 35 |
| 18.17 | 4.88 | 32 |
| 18.32 | 4.84 | 28 |
| 18.72 | 4.74 | 8 |
| 19.16 | 4.63 | 30 |
| 19.96 | 4.45 | 26 |
| 20.28 | 4.37 | 56 |
| 20.60 | 4.31 | 7 |
| 21.14 | 4.20 | 84 |
| 21.64 | 4.10 | 100 |
| 22.33 | 3.98 | 15 |
| 23.23 | 3.83 | 41 |
| 24.06 | 3.70 | 4 |
| 24.51 | 3.63 | 15 |
| 24.93 | 3.57 | 26 |
| 25.89 | 3.44 | 23 |
| 26.21 | 3.40 | 11 |
| 26.84 | 3.32 | 8 |
| 27.66 | 3.22 | 38 |
| 27.96 | 3.19 | 9 |
| 28.26 | 3.16 | 5 |
| 28.44 | 3.14 | 6 |
| 28.75 | 3.10 | 6 |
| 29.18 | 3.06 | 19 |

Even more specifically, said crystalline complex is characterised by an X-ray powder diffraction pattern, made using CuK_{α1} radiation, which comprises peaks at degrees 2Θ (±0.1 degrees 2Θ) as shown in Figure 1.

Furthermore said crystalline complex of the compound A with L-proline is characterised by a melting point of above 89°C, in particular in a range from about 89°C to about 115°C, more preferably in a range from about 89°C to about 110°C (determined via DSC; evaluated as onset-temperature; heating rate 10 K/min). It can be observed that this crystalline complex melts under dehydration. The obtained DSC curve is shown in Figure 2.

Said crystalline complex of the compound A with L-proline shows a weight loss by thermal gravimetry (TG). The observed weight loss indicates that the crystalline form contains water which may be bound by adsorption and/or may be part of the crystalline lattice, i.e the crystalline form may be present as a crystalline hydrate. The content of water in the crystalline form lies in the range from 0 to about 10 weight-%, in particular 0 to about 5 weight-%, even more preferably from about 1.5 to about 5 weight-%. The dotted line in Figure 2 depicts a weight loss of between 2.8 and 3.8 % of water. From the observed weight loss a stoichiometry close to a monohydrate can be estimated.

Said crystalline complex has advantageous physico-chemical properties which are beneficial in the preparation of a pharmaceutical composition. In particular the crystalline complex has a high physical and chemical stability under various environmental conditions and during the production of a medicament. For example the crystals can be obtained in a shape and particle size which are particular suitable in a production method for solid pharmaceutical formulations. In addition the crystals show a high mechanical stability that allows grinding of the crystals. Furthermore the crystalline complex does not show a high tendency to absorb moisture and is chemically stable, i.e. the crystalline complex allows the production of a solid pharmaceutical formulation with a long shelf life. On the other hand the crystalline complex has a favorably high solubility over a wide pH-range which is advantageous in solid pharmaceutical formulations for oral administration.

The X-ray powder diffraction patterns are recorded, within the scope of the present invention, using a STOE - STADI P-diffractometer in transmission mode fitted with a location-sensitive detector (OED) and a Cu-anode as X-ray source (CuK_{α1} radiation, λ = 1.54056 Å , 40kV, 40mA). In Table 1 the values "2Θ [°]" denote the angle of diffraction in degrees and the values "d [Å]" denote the specified distances in Å between the lattice planes. The intensity shown in Figure 1 is given in units of cps (counts per second).

In order to allow for experimental error, the above described 2 Θ values should be considered accurate to ± 0.1 degrees 2 Θ, in particular ± 0.05 degrees 2 Θ. That is to say, when assessing whether a given sample of crystals of the compound A is the crystalline form in accordance with the invention, a 2 Θ value which is experimentally observed for the sample should be considered identical with a characteristic value described above if it falls within ± 0.1 degrees 2 Θ of the characteristic value, in particular if it falls within ± 0.05 degrees 2 Θ of the characteristic value.

The melting point is determined by DSC (Differential Scanning Calorimetry) using a DSC 821 (Mettler Toledo). The weight loss is determined by thermal gravimetry (TG) using a TGA 851 (Mettler Toledo).

The compound A may be synthesized by methods as specifically and/or generally described or cited in the international application WO 2007/128749. Furthermore the biological properties of the compound A may be investigated as it is described in the international application WO 2007/128749.

The crystalline complex in accordance with the invention is preferably employed as drug active substance in substantially pure form, that is to say, essentially free of other crystalline forms of the compound A. Nevertheless, the invention also embraces the crystalline complex in admixture with another crystalline form or forms. Should the drug active substance be a mixture of crystalline forms, it is preferred that the substance comprises at least 50%-weight, even more preferably at least 90%-weight, most preferably at least 95%-weight of the crystalline complex as described herein.

In view of their ability to inhibit the SGLT activity, the crystalline complex according to the invention is suitable for the use in the treatment and/or preventive treatment of conditions or diseases which may be affected by the inhibition of the SGLT activity, particularly the SGLT-2 activity. The crystalline complex according to the invention is also suitable for the preparation of pharmaceutical compositions for the treatment and/or preventive treatment of conditions or diseases which may be affected by the inhibition of the SGLT activity, particularly the SGLT-2 activity. Therefore, the crystalline complex according to the invention is suitable for the use in the treatment of type 2 diabetes mellitus in humans.

The dosage required to achieve the corresponding activity for treatment or prevention usually depends on the patient, the nature and gravity of the illness or condition and the method and frequency of administration and is for the patient's doctor to decide. Expediently, the dosage may be from 1 to 100 mg by intravenous route, and 1 to 1000 mg by oral route, in each case administered 1 to 4 times a day. For this purpose, the pharmaceutical compositions according to this invention preferably comprise the crystalline complex according to the invention together with one or more inert conventional carriers and/or diluents. Such pharmaceutical compositions may be formulated as conventional galenic preparations such as plain or coated tablets, capsules, powders, suspensions or suppositories.

In the foregoing and following text, H atoms of hydroxyl groups are not explicitly shown in every case in structural formulae. The following example of synthesis serves to illustrate a method of preparing the compound A and its crystalline complex with L-proline as a natural amino acid. It is to be regarded only as a possible method described by way of example, without restricting the invention to its contents. The terms "room temperature" and "ambient temperature" are used interchangeably and denote temperatures of about 20°C. The following abbreviations are used:
- DMF: dimethylformamide
- NMP: *N*-methyl-2-pyrrolidone
- THF: tetrahydrofuran

### Preparation of the starting compounds:

### Example I

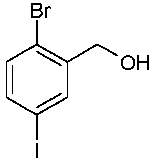

### 4-Bromo-3-hydroxymethyl-1-iodo-benzene

Oxalyl chloride (13.0 mL) is added to an ice-cold solution of 2-bromo-5-iodo-benzoic acid (49.5 g) in CH₂Cl₂ (200 mL). DMF (0.2 mL) is added and the solution is stirred at room temperature for 6 h. Then, the solution is concentrated under reduced pressure and the residue is dissolved in THF (100 mL). The resulting solution is cooled in an ice-bath and LiBH₄ (3.4 g) is added in portions. The cooling bath is removed and the mixture is stirred at room temperature for 1 h. The reaction mixture is diluted with THF and treated with 0.1 M hydrochloric acid. Then, the organic layer is separated and the aqueous layer is extracted with ethyl acetate. The combined organic layers are dried (Na₂SO₄) and the solvent is evaporated under reduced pressure to give the crude product.

Yield: 47.0 g (99% of theory)

### Example II

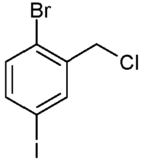

### 4-Bromo-3-chloromethyl-1-iodo-benzene

Thionyl chloride (13 mL) is added to a suspension of 4-bromo-3-hydroxymethyl-1-iodobenzene (47.0 g) in dichloromethane (100 mL) containing DMF (0.1 mL). The mixture is stirred at ambient temperature for 3 h. Then, the solvent and the excess reagent is removed under reduced pressure. The residue is triturated with methanol and dried.

Yield: 41.0 g (82% of theory)

### Example III

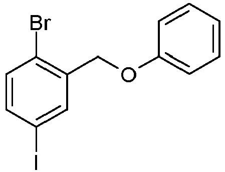

### 4-Bromo-1-iodo-3-phenoxymethyl-benzene

Phenol (13 g) dissolved in 4 M KOH solution (60 mL) is added to 4-bromo-3-chloromethyl-1-iodo-benzene (41.0 g) dissolved in acetone (50 mL). NaI (0.5 g) is added and the resulting mixture is stirred at 50 °C overnight. Then, water is added and the resulting mixture is extracted with ethyl acetate. The combined extracts are dried (Na₂SO₄) and the solvent is evaporated under reduced pressure. The residue is purified by chromatography on silica gel (cyclohexane/ethyl acetate 19:1).

Yield: 38.0 g (79% of theory)

### Example IV

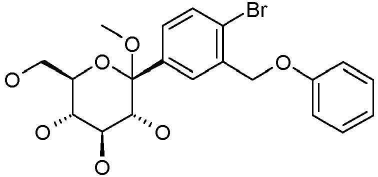

### 1-Bromo-4-(1-methoxy-D-glucopyranos-1-yl)-2-(phenoxymethyl)-benzene

A 2 M solution of iPrMgCl in THF (11 mL) is added to dry LiCl (0.47 g) suspended in THF (11 mL). The mixture is stirred at room temperature until all the LiCl is dissolved. This solution is added dropwise to a solution of 4-bromo-1-iodo-3-phenoxymethyl-benzene (8.0 g) in tetrahydrofuran (40 mL) cooled to -60 °C under argon atmosphere. The solution is warmed to -40 °C and then 2,3,4,6-tetrakis-O-(trimethylsilyl)-D-glucopyranone (10.7 g, 90% pure) in tetrahydrofuran (5 mL) is added. The resulting solution is warmed to -5 °C in the cooling bath and stirred for another 30 min at this temperature. Aqueous NH₄Cl solution is added and the resultant mixture is extracted with ethyl acetate. The combined organic extracts are dried over sodium sulfate and the solvent is removed under reduced pressure. The residue is dissolved in methanol (80 mL) and treated with methanesulfonic acid (0.6 mL) to produce the more stable anomer solely. After stirring the reaction solution at 35-40 °C overnight, the solution is neutralized with solid NaHCO₃ and the methanol is removed under reduced pressure. The remainder is diluted with aqueous NaHCO₃ solution and the resulting mixture is extracted with ethyl acetate. The combined extracts are dried over sodium sulfate and the solvent is evaporated to yield the crude product that is submitted to reduction without further purification.

Yield: 7.8 g (93% of theory)

### Example V

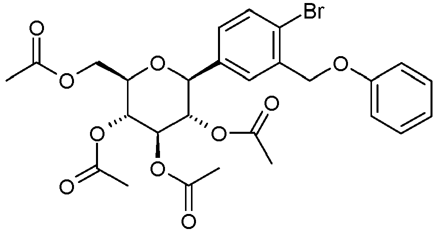

### 1-Bromo-4-(2,3,4,6-tetra-O-acetyl-D-glucopyranos-1-yl)-2-(phenoxymethyl)-benzene

Boron trifluoride diethyletherate (4.9 mL) is added to a solution of 1-bromo-4-(1-methoxy-D-glucopyranos-1-yl)-2-(phenoxymethyl)-benzene (8.7 g) and triethylsilane (9.1 mL) in dichloromethane (35 mL) and acetonitrile (50 mL) cooled to -20 °C at such a rate that the temperature maintains below -10 °C. The resultant solution is warmed to 0 °C over a period of 1.5 h and then treated with aqueous sodium hydrogen carbonate solution. The resulting mixture is stirred for 0.5 h, the organic solvent is removed and the residue is extracted with ethyl acetate. The combined organic layers are dried over sodium sulfate and the solvent is removed. The residue is taken up in dichloromethane (50 mL) and pyridine (9.4 mL), acetic anhydride (9.3 mL) and 4-dimethylaminopyridine (0.5 g) are added in succession to the solution. The solution is stirred for 1.5 h at ambient temperature and then diluted with dichloromethane. This solution is washed twice with 1 M hydrochloric acid and dried over sodium sulfate. After the solvent is removed, the residue is recrystallized from ethanol to furnish the product as a colorless solid.

Yield: 6.78 g (60% of theory)
Mass spectrum (ESI⁺): m/z = 610/612 (Br) [M+NH₄]⁺

### Example VI

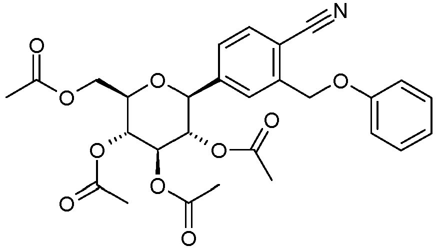

### 2-(Phenoxymethyl)-4-(2,3,4,6-tetra-O-acetyl-D-glucopyranos- 1-yl)-benzonitrile

A flask charged with zinc cyanide (1.0 g), zinc (30 mg), Pd₂(dibenzylideneacetone)₃*CHCl₃ (141 mg) and tri-tert-butylphosphonium tetrafluoroborate (111 mg) is flushed with argon. Then a solution of 1-bromo-4-(2,3,4,6-tetra-O-acetyl-D-glucopyranos-1-yl)-2-(phenoxymethyl)-benzene (5.4 g) in NMP (12 mL) is added and the resulting mixture is stirred at room temperature for 18 h. After dilution with ethyl acetate, the mixture is filtered and the filtrate is washed with aqueous sodium hydrogen carbonate solution. The organic phase is dried (sodium sulfate) and the solvent is removed. The residue is recrystallized from ethanol.

Yield: 4.10 g (84% of theory)
Mass spectrum (ESI⁺): m/z = 557 [M+NH₄]⁺

Alternatively, the compound described above is synthesized starting from 1-bromo-4-(2,3,4,6-tetra-O-acetyl-D-glucopyranos-1-yl)-2-(phenoxymethyl)-benzene using copper(I) cyanide (2 equivalents) in NMP at 210 °C.

### Example VII

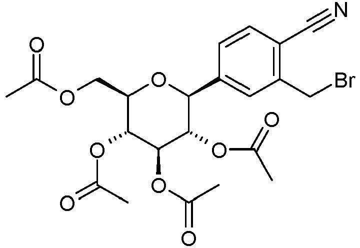

### 2-Bromomethyl-4-(2,3,4,6-tetra-O-acetyl-D-glucopyranos-1-yl)-benzonitrile

A 33% solution of hydrobromic acid in acetic acid (15 mL) is added to a solution of 2-phenyloxymethyl-4-(2,3,4,6-tetra-O-acetyl-D-glucopyranos-1-yl)-benzonitrile (0.71 g) and acetic anhydride (0.12 mL) in acetic acid (10 ml). The resulting solution is stirred at 55 °C for 6 h and then cooled in an ice-bath. The reaction mixture is neutralized with chilled aqueous potassium carbonate solution, and the resultant mixture is extracted with ethyl acetate. The combined organic extracts are dried over sodium sulfate and the solvent is removed under reduced pressure. The residue is taken up in ethyl acetate/cyclohexane (1:5), and the precipitate is separated by filtration and dried at 50 °C to give the pure product.

Yield: 0.52 g (75% of theory)
Mass spectrum (ESI⁺): m/z = 543/545 (Br) [M+NH₄]⁺

### Example VIII

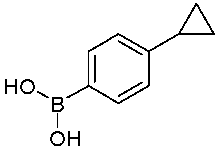

### 4-Cyclopropyl-phenylboronic acid

2.5 M solution of nButyllithium in hexane (14.5 mL) is added dropwise to 1-bromo-4-cyclopropyl-benzene (5.92 g) dissolved in THF (14 mL) and toluene (50 mL) and chilled to - 70 °C. The resultant solution is stirred at -70 °C for 30 min before triisopropyl borate (8.5 mL) is added. The solution is warmed to -20 °C and then treated with 4 M aqueous hydrochloric acid (15.5 mL). The reaction mixture is further warmed to room temperature and then the organic phase is separated. The aqueous phase is extracted with ethyl acetate and the combined organic phases are dried (sodium sulfate). The solvent is evaporated and the residue is washed with a mixture of ether and cyclohexane to give the product as a colorless solid.

Yield: 2.92 g (60% of theory)
Mass spectrum (ESI⁻): m/z = 207 (CI) [M+HCOO]⁻

### Preparation of the compound A:

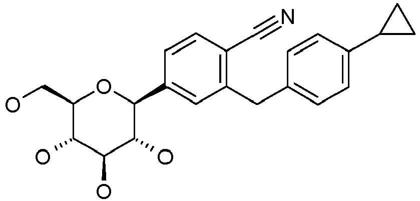

### 1-Cyano-2-(4-cyclopropyl-benzyl)-4-(3-D-glucopyranos-1-yl)-benzene

An Ar filled flask is charged with 2-bromomethyl-4-(2,3,4,6-tetra-O-acetyl-D-glucopyranos-1-yl)-benzonitrile (1.60 g), 4-cyclopropyl-phenylboronic acid (1.0 g), potassium carbonate (1.85 g) and a degassed 3:1 mixture of acetone and water (22 mL). The mixture is stirred at room temperature for 5 min, before it is cooled in an ice-bath. Then palladium dichloride (30 mg) is added and the reaction mixture is stirred for 16 h at ambient temperature. The mixture is then diluted with brine and extracted with ethyl acetate. The combined extracts are dried over sodium sulfate and the solvent is removed under reduced pressure. The residue is dissolved in methanol (20 mL) and treated with 4 M aqueous potassium hydroxide solution (4 mL). The resulting solution is stirred at ambient temperature for 1 h and then neutralized with 1 M hydrochloric acid. The methanol is evaporated, and the residue is diluted with brine and extracted with ethyl acetate. The organic extracts collected are dried over sodium sulfate, and the solvent is removed. The residue is chromatographed on silica gel (dichloromethane/methanol 1:0 -> 8:1).

Yield: 0.91 g (76% of theory )
Mass spectrum (ESI⁺): m/z = 413 [M+NH₄]⁺

### Preparation of the crystalline complex (1 : 1) with L-proline:

L-proline (0.34 g) dissolved in 2.1 mL of a mixture of ethanol and water (volume ratio 10:1) is added to a solution of 1-cyano-2-(4-cyclopropyl-benzyl)-4-(ß-D-glucopyranos-1-yl)-benzene (1.17 g, obtained as described above) dissolved in 2 mL ethanol. The resulting solution is allowed to stand at ambient temperature. After about 16 h the crystalline complex is isolated as white crystals by filtration. If necessary the crystallisation may be initiated by scratching with a glass rod or metal spatula for example or by inoculating with seed crystals. Residual solvent is removed by storing the crystals at slightly elevated temperature (30 to 50 °C) under vacuum for about 4 h to yield 1.27 g of the crystalline 1:1 complex of L-proline and 1-cyano-2-(4-cyclopropyl-benzyl)-4-(β-D-glucopyranos-1-yl)-benzene.

Several batches of the crystalline complex according to the above preparation are obtained. The X-ray powder diffraction patterns coincide. The melting points are determined via DSC and evaluated as onset-temperature. Examples of melting points are approximately 89°C, 90°C, 92°C, 101°C and 110°C. The X-ray powder diffraction pattern as contained in Table 1 and as depicted in Fig. 1 and the DSC and TG diagram in Fig. 2 correspond to a batch with a melting point of approximately 90°C.

## Claims

1. A mixture of crystalline forms comprising at least one crystalline complex in admixture with another crystalline form or forms of 1-cyano-2-(4-cyclopropyl-benzyl)-4-(ß-D-glucopyranos-1-yl)-benzene, wherein the crystalline complex is a crystalline complex between 1-cyano-2-(4-cyclopropyl-benzyl)-4-(ß-D-glucopyranos-1-yl)-benzene and one or more natural amino acids:
(i) **characterized in that** the natural amino acid is L-proline,
(ii) **characterized in that** the crystalline complex is a complex (1:1) between 1-cyano-2-(4-cyclopropyl-benzyl)-4-(ß-D-glucopyranos-1-yl)-benzene and L-proline,
(iii) **characterized in that** the crystalline complex is a crystalline hydrate, and
(iv) **characterized in that** the molar ratio of the crystalline complex of 1-cyano-2-(4-cyclopropyl-benzyl)-4-(ß-D-glucopyranos-1-yl)-benzene and L-proline with crystal water is 1:1.

2. The mixture of crystalline forms according to claim 1, wherein the crystalline complex is **characterized by** an X-ray powder diffraction pattern that comprises peaks at 20.28, 21.14 and 21.64 degrees 2Θ (±0.1 degrees 2Θ), wherein said X-ray powder diffraction pattern is made using CuK_{α1} radiation.

3. The mixture of crystalline forms according to any one of claims 1 to 2, wherein the X-ray powder diffraction pattern of the crystalline complex further comprises peaks at 4.99 and 23.23 degrees 2Θ (±0.1 degrees 2Θ), wherein said X-ray powder diffraction pattern is made using CuK_{α1} radiation.

4. The mixture of crystalline forms according to any one of claims 1 to 3, wherein the X-ray powder diffraction pattern of the crystalline complex further comprises peaks at 17.61, 17.77 and 27.66 degrees 2Θ (±0.1 degrees 2Θ), wherein said X-ray powder diffraction pattern is made using CuK_{α1} radiation.

5. The mixture of crystalline forms according to any one of claims 1 to 4, wherein the X-ray powder diffraction pattern of the crystalline complex comprises peaks at 4.99, 15.12, 17.61, 17.77, 18.17, 20.28, 21.14, 21.64, 23.23 and 27.66 degrees 2Θ (±0.1 degrees 2Θ), wherein said X-ray powder diffraction pattern is made using CuK_{α1} radiation.

6. The mixture of crystalline forms according to any one of claims 1 to 5, wherein the crystalline complex is **characterised by** an X-ray powder diffraction pattern, made using CuK_{α1} radiation, which comprises peaks at degrees 2Θ (±0.1 degrees 2Θ) as follows:
| **2 Θ [°]** | **d-value [Å]** | **Intensity I/I₀ [%]** |
|---|---|---|
| 4.99 | 17.68 | 39 |
| 7.01 | 12.61 | 6 |
| 8.25 | 10.70 | 11 |
| 9.95 | 8.88 | 12 |
| 13.15 | 6.73 | 30 |
| 13.33 | 6.64 | 10 |
| 14.08 | 6.28 | 4 |
| 15.12 | 5.85 | 32 |
| 16.40 | 5.40 | 12 |
| 16.49 | 5.37 | 13 |
| 17.11 | 5.18 | 6 |
| 17.61 | 5.03 | 32 |
| 17.77 | 4.99 | 35 |
| 18.17 | 4.88 | 32 |
| 18.32 | 4.84 | 28 |
| 18.72 | 4.74 | 8 |
| 19.16 | 4.63 | 30 |
| 19.96 | 4.45 | 26 |
| 20.28 | 4.37 | 56 |
| 20.60 | 4.31 | 7 |
| 21.14 | 4.20 | 84 |
| 21.64 | 4.10 | 100 |
| 22.33 | 3.98 | 15 |
| 23.23 | 3.83 | 41 |
| 24.06 | 3.70 | 4 |
| 24.51 | 3.63 | 15 |
| 24.93 | 3.57 | 26 |
| 25.89 | 3.44 | 23 |
| 26.21 | 3.40 | 11 |
| 26.84 | 3.32 | 8 |
| 27.66 | 3.22 | 38 |
| 27.96 | 3.19 | 9 |
| 28.26 | 3.16 | 5 |
| 28.44 | 3.14 | 6 |
| 28.75 | 3.10 | 6 |
| 29.18 | 3.06 | 19 |

7. The mixture of crystalline forms according to any one of claims 1 to 6, wherein said crystalline complex is **characterised by** an X-ray powder diffraction pattern, made using CuK_{α1} radiation, which comprises peaks at degrees 2Θ (±0.1 degrees 2Θ) as shown in Figure 1.

8. The mixture of crystalline forms according to any one of claims 1 to 7 comprising at least 50%-weight, preferably at least 90%-weight, more preferably at least 95%-weight of the crystalline complex as claimed in any one of claims 1 to 7.

9. Use of the mixture of crystalline forms according to any one of claims 1 to 8 for the preparation of a pharmaceutical composition.

10. The mixture of crystalline forms according to any one of claims 1 to 8 for use in the preparation of a pharmaceutical composition, preferably for use in the preparation of a pharmaceutical composition for the treatment or prevention of diseases or conditions which can be influenced by inhibiting the sodium-dependent glucose cotransporter SGLT, more preferably the sodium-dependent glucose cotransporter SGLT2.

11. The mixture of crystalline forms according to any one of claims 1 to 8 for use as a medicament.

12. The mixture of crystalline forms according to any one of claims 1 to 8 for use in the treatment or prevention of diseases or conditions which can be influenced by inhibiting the sodium-dependent glucose cotransporter SGLT, more preferably the sodium-dependent glucose cotransporter SGLT2.

13. Use of a crystalline complex between 1-cyano-2-(4-cyclopropyl-benzyl)-4-(ß-D-glucopyranos-1-yl)-benzene and one or more natural amino acids for the preparation of a pharmaceutical composition,
(i) **characterized in that** the natural amino acid is L-proline, and
(ii) **characterized in that** the crystalline complex is a complex (1:1) between 1-cyano-2-(4-cyclopropyl-benzyl)-4-(ß-D-glucopyranos-1-yl)-benzene and L-proline, and
(iii) **characterized in that** the crystalline complex is a crystalline hydrate, and
(iv) **characterized in that** the molar ratio of the crystalline complex of 1-cyano-2-(4-cyclopropyl-benzyl)-4-(ß-D-glucopyranos-1-yl)-benzene and L-proline with crystal water is 1:1.

14. The crystalline complex as defined in claim 13 for use in the preparation of a pharmaceutical composition, preferably for use in the preparation of a pharmaceutical composition for the treatment or prevention of diseases or conditions which can be influenced by inhibiting the sodium-dependent glucose cotransporter SGLT, more preferably the sodium-dependent glucose cotransporter SGLT2.

## Patentansprüche

1. Gemisch von kristallinen Formen, umfassend mindestens einen kristallinen Komplex in Mischung mit einer anderen kristallinen Form bzw. anderen kristallinen Formen von 1-Cyano-2-(4-cyclopropylbenzyl)-4-(ß-D-glucopyranos-1-yl)benzol, wobei es sich bei dem kristallinen Komplex um einen kristallinen Komplex zwischen 1-Cyano-2-(4-cyclopropylbenzyl)-4-(ß-D-glucopyranos-1-yl)benzol und einer oder mehreren natürlichen Aminosäuren handelt:
(i) **dadurch gekennzeichnet, dass** es sich bei der natürlichen Aminosäure um L-Prolin handelt,
(ii) **dadurch gekennzeichnet, dass** es sich bei dem kristallinen Komplex um einen Komplex (1:1) zwischen 1-Cyano-2-(4-cyclopropylbenzyl)-4-(ß-D-glucopyranos-1-yl)benzol und L-Prolin handelt,
(iii) **dadurch gekennzeichnet, dass** es sich bei dem kristallinen Komplex um ein kristallines Hydrat handelt, und
(iv) **dadurch gekennzeichnet, dass** das Molverhältnis des kristallinen Komplexes von 1-Cyano-2-(4-cyclopropylbenzyl)-4-(ß-D-glucopyranos-1-yl)benzol und L-Prolin mit Kristallwasser 1:1 beträgt.

2. Gemisch von kristallinen Formen nach Anspruch 1, wobei der kristalline Komplex durch ein Röntgenpulverbeugungsmuster gekennzeichnet ist, das Peaks bei 20,28, 21,14 und 21,64 Grad 2Θ (±0,1 Grad 2Θ) umfasst, wobei das Röntgenpulverbeugungsmuster unter Verwendung von CuK_{α1}-Strahlung erstellt wird.

3. Gemisch von kristallinen Formen nach einem der Ansprüche 1 bis 2, wobei das Röntgenpulverbeugungsmuster des kristallinen Komplexes ferner Peaks bei 4,99 und 23,23 Grad 2Θ (±0,1 Grad 2Θ) umfasst, wobei das Röntgenpulverbeugungsmuster unter Verwendung von CuK_{α1}-Strahlung erstellt wird.

4. Gemisch von kristallinen Formen nach einem der Ansprüche 1 bis 3, wobei das Röntgenpulverbeugungsmuster des kristallinen Komplexes ferner Peaks bei 17,61, 17,77 und 27,66 Grad 2Θ (±0,1 Grad 2Θ) umfasst, wobei das Röntgenpulverbeugungsmuster unter Verwendung von CuK_{α1}-Strahlung erstellt wird.

5. Gemisch von kristallinen Formen nach einem der Ansprüche 1 bis 4, wobei das Röntgenpulverbeugungsmuster des kristallinen Komplexes Peaks bei 4,99, 15,12, 17,61, 17,77, 18,17, 20,28, 21,14, 21,64, 23,23 und 27,66 Grad 2Θ (±0,1 Grad 2Θ) umfasst, wobei das Röntgenpulverbeugungsmuster unter Verwendung von CuK_{α1}-Strahlung erstellt wird.

6. Gemisch von kristallinen Formen nach einem der Ansprüche 1 bis 5, wobei der kristalline Komplex durch ein unter Verwendung von CuK_{α1}-Strahlung erstelltes Röntgenpulverbeugungsmuster gekennzeichnet ist, das Peaks bei Grad 2Θ (±0,1 Grad 2Θ) wie folgt umfasst:
| 2Θ [°] | d-Wert [Å] | Intensität I/I₀ [%] |
|---|---|---|
| 4,99 | 17,68 | 39 |
| 7,01 | 12,61 | 6 |
| 8,25 | 10,70 | 11 |
| 9,95 | 8,88 | 12 |
| 13,15 | 6,73 | 30 |
| 13,33 | 6,64 | 10 |
| 14,08 | 6,28 | 4 |
| 15,12 | 5,85 | 32 |
| 16,40 | 5,40 | 12 |
| 16,49 | 5,37 | 13 |
| 17,11 | 5,18 | 6 |
| 17,61 | 5,03 | 32 |
| 17,77 | 4,99 | 35 |
| 18,17 | 4,88 | 32 |
| 18,32 | 4,84 | 28 |
| 18,72 | 4,74 | 8 |
| 19,16 | 4,63 | 30 |
| 19,96 | 4,45 | 26 |
| 20,28 | 4,37 | 56 |
| 20,60 | 4,31 | 7 |
| 21,14 | 4,20 | 84 |
| 21,64 | 4,10 | 100 |
| 22,33 | 3,98 | 15 |
| 23,23 | 3,83 | 41 |
| 24,06 | 3,70 | 4 |
| 24,51 | 3,63 | 15 |
| 24,93 | 3,57 | 26 |
| 25,89 | 3,44 | 23 |
| 26,21 | 3,40 | 11 |
| 26,84 | 3,32 | 8 |
| 27,66 | 3,22 | 38 |
| 27,96 | 3,19 | 9 |
| 28,26 | 3,16 | 5 |
| 28,44 | 3,14 | 6 |
| 28,75 | 3,10 | 6 |
| 29,18 | 3,06 | 19 |

7. Gemisch von kristallinen Formen nach einem der Ansprüche 1 bis 6, wobei der kristalline Komplex durch ein unter Verwendung von CuK_{α1}-Strahlung erstelltes Röntgenpulverbeugungsmuster gekennzeichnet ist, das Peaks bei Grad 2Θ (±0,1 Grad 2Θ) gemäß der Darstellung in Figur 1 umfasst.

8. Gemisch von kristallinen Formen nach einem der Ansprüche 1 bis 7, umfassend mindestens 50 Gew.-%, vorzugsweise mindestens 90 Gew.-%, weiter bevorzugt mindestens 95 Gew.-%, des kristallinen Komplexes gemäß einem der Ansprüche 1 bis 7.

9. Verwendung des Gemischs von kristallinen Formen nach einem der Ansprüche 1 bis 8 zur Herstellung einer pharmazeutischen Zusammensetzung.

10. Gemisch von kristallinen Formen nach einem der Ansprüche 1 bis 8 zur Verwendung bei der Herstellung einer pharmazeutischen Zusammensetzung, vorzugsweise zur Verwendung bei der Herstellung einer pharmazeutischen Zusammensetzung zur Behandlung oder Prävention von Krankheiten oder Leiden, die durch Hemmung des natriumabhängigen Glucose-Cotransporters SGLT, weiter bevorzugt des natriumabhängigen Glucose-Cotransporters SGLT2, beeinflusst werden können.

11. Gemisch von kristallinen Formen nach einem der Ansprüche 1 bis 8 zur Verwendung als Medikament.

12. Gemisch von kristallinen Formen nach einem der Ansprüche 1 bis 8 zur Verwendung bei der Behandlung oder Prävention von Krankheiten oder Leiden, die durch Hemmung des natriumabhängigen Glucose-Cotransporters SGLT, weiter bevorzugt des natriumabhängigen Glucose-Cotransporters SGLT2, beeinflusst werden können.

13. Verwendung eines kristallinen Komplexes zwischen 1-Cyano-2-(4-cyclopropylbenzyl)-4-(ß-D-glucopyranos-1-yl)benzol und einer oder mehreren natürlichen Aminosäuren zur Herstellung einer pharmazeutischen Zusammensetzung,
(i) **dadurch gekennzeichnet, dass** es sich bei der natürlichen Aminosäure um L-Prolin handelt, und
(ii) **dadurch gekennzeichnet, dass** es sich bei dem kristallinen Komplex um einen Komplex (1:1) zwischen 1-Cyano-2-(4-cyclopropylbenzyl)-4-(ß-D-glucopyranos-1-yl)benzol und L-Prolin handelt, und
(iii) **dadurch gekennzeichnet, dass** es sich bei dem kristallinen Komplex um ein kristallines Hydrat handelt, und
(iv) **dadurch gekennzeichnet, dass** das Molverhältnis des kristallinen Komplexes von 1-Cyano-2-(4-cyclopropylbenzyl)-4-(ß-D-glucopyranos-1-yl)benzol und L-Prolin mit Kristallwasser 1:1 beträgt.

14. Kristalliner Komplex gemäß Anspruch 13 zur Verwendung bei der Herstellung einer pharmazeutischen Zusammensetzung, vorzugsweise zur Verwendung bei der Herstellung einer pharmazeutischen Zusammensetzung zur Behandlung oder Prävention von Krankheiten oder Leiden, die durch Hemmung des natriumabhängigen Glucose-Cotransporters SGLT, weiter bevorzugt des natriumabhängigen Glucose-Cotransporters SGLT2, beeinflusst werden können.

## Revendications

1. Mélange de formes cristallines comprenant au moins un complexe cristallin en mélange avec une autre forme cristalline ou d'autres formes cristallines de 1-cyano-2-(4-cyclopropyl-benzyl)-4-(ß-D-glucopyranos-1-yl)-benzène, dans lequel le complexe cristallin est un complexe cristallin entre le 1-cyano-2-(4-cyclopropyl-benzyl)-4-(ß-D-glucopyranos-1-yl)-benzène et un ou plusieurs acides aminés naturels :
(i) **caractérisé en ce que** l'acide aminé naturel est la L-proline,
(ii) **caractérisé en ce que** le complexe cristallin est un complexe (1:1) entre le 1-cyano-2-(4-cyclopropyl-benzyl)-4-(ß-D-glucopyranos-1-yl)-benzène et la L-proline,
(iii) **caractérisé en ce que** le complexe cristallin est un hydrate cristallin, et
(iv) **caractérisé en ce que** le rapport molaire du complexe cristallin de 1-cyano-2-(4-cyclopropyl-benzyl)-4-(ß-D-glucopyranos-1-yl)-benzène et de L-proline avec l'eau cristalline est de 1:1.

2. Mélange de formes cristallines selon la revendication 1, dans lequel le complexe cristallin est **caractérisé par** un diagramme de diffraction des rayons X sur poudre qui comprend des pics à 20,28, 21,14 et 21,64 degrés 2Θ (± 0,1 degré 2Θ), dans lequel ledit diagramme de diffraction des rayons X sur poudre est réalisé en utilisant un rayonnement CuK_{α1}.

3. Mélange de formes cristallines selon l'une quelconque des revendications 1 à 2, dans lequel le diagramme de diffraction des rayons X sur poudre du complexe cristallin comprend en outre des pics à 4,99 et 23,23 degrés 2Θ (± 0,1 degré 2Θ), dans lequel ledit diagramme de diffraction des rayons X sur poudre est réalisé en utilisant un rayonnement CuK_{α1}.

4. Mélange de formes cristallines selon l'une quelconque des revendications 1 à 3, dans lequel le diagramme de diffraction des rayons X sur poudre du complexe cristallin comprend en outre des pics à 17,61, 17,77 et 27,66 degrés 2Θ (± 0,1 degré 2Θ), dans lequel ledit diagramme de diffraction des rayons X sur poudre est réalisé en utilisant un rayonnement CuK_{α1}.

5. Mélange de formes cristallines selon l'une quelconque des revendications 1 à 4, dans lequel le diagramme de diffraction des rayons X sur poudre du complexe cristallin comprend des pics à 4,99, 15,12, 17,61, 17,77, 18,17, 20,28, 21,14, 21,64, 23,23 et 27,66 degrés 2Θ (± 0,1 degré 2Θ), dans lequel ledit diagramme de diffraction des rayons X sur poudre est réalisé en utilisant un rayonnement CuK_{α1}.

6. Mélange de formes cristallines selon l'une quelconque des revendications 1 à 5, dans lequel le complexe cristallin est **caractérisé par** un diagramme de diffraction des rayons X sur poudre, réalisé en utilisant un rayonnement CuK_{α1}, qui comprend des pics à des degrés 2Θ (± 0,1 degré 2Θ) comme suit :
| 2Θ [°] | Valeur d [Å] | Intensité I/I₀ [%] |
|---|---|---|
| 4,99 | 17,68 | 39 |
| 7,01 | 12,61 | 6 |
| 8,25 | 10,70 | 11 |
| 9,95 | 8,88 | 12 |
| 13,15 | 6,73 | 30 |
| 13,33 | 6,64 | 10 |
| 14,08 | 6,28 | 4 |
| 15,12 | 5,85 | 32 |
| 16,40 | 5,40 | 12 |
| 16,49 | 5,37 | 13 |
| 17,11 | 5,18 | 6 |
| 17,61 | 5,03 | 32 |
| 17,77 | 4,99 | 35 |
| 18,17 | 4,88 | 32 |
| 18,32 | 4,84 | 28 |
| 18,72 | 4,74 | 8 |
| 19,16 | 4,63 | 30 |
| 19,96 | 4,45 | 26 |
| 20,28 | 4,37 | 56 |
| 20,60 | 4,31 | 7 |
| 21,14 | 4,20 | 84 |
| 21,64 | 4,10 | 100 |
| 22,33 | 3,98 | 15 |
| 23,23 | 3,83 | 41 |
| 24,06 | 3,70 | 4 |
| 24,51 | 3,63 | 15 |
| 24,93 | 3,57 | 26 |
| 25,89 | 3,44 | 23 |
| 26,21 | 3,40 | 11 |
| 26,84 | 3,32 | 8 |
| 27,66 | 3,22 | 38 |
| 27,96 | 3,19 | 9 |
| 28,26 | 3,16 | 5 |
| 28,44 | 3,14 | 6 |
| 28,75 | 3,10 | 6 |
| 29,18 | 3,06 | 19 |

7. Mélange de formes cristallines selon l'une quelconque des revendications 1 à 6, dans lequel ledit complexe cristallin est **caractérisé par** un diagramme de diffraction des rayons X sur poudre, réalisé en utilisant un rayonnement CuK_{α1}, qui comprend des pics à des degrés 2Θ (± 0,1 degré 2Θ) comme représenté sur la Figure 1.

8. Mélange de formes cristallines selon l'une quelconque des revendications 1 à 7 comprenant au moins 50 % en poids, préférablement au moins 90 % en poids, plus préférablement au moins 95 % en poids du complexe cristallin selon l'une quelconque des revendications 1 à 7.

9. Utilisation du mélange de formes cristallines selon l'une quelconque des revendications 1 à 8 pour la préparation d'une composition pharmaceutique.

10. Mélange de formes cristallines selon l'une quelconque des revendications 1 à 8, destiné à être utilisé dans la préparation d'une composition pharmaceutique, préférablement destiné à être utilisé dans la préparation d'une composition pharmaceutique pour le traitement ou la prévention de maladies ou d'affections qui peuvent être influencées par l'inhibition du cotransporteur de glucose dépendant du sodium SGLT, plus préférablement du cotransporteur de glucose dépendant du sodium SGLT2.

11. Mélange de formes cristallines selon l'une quelconque des revendications 1 à 8, destiné à être utilisé comme médicament.

12. Mélange de formes cristallines selon l'une quelconque des revendications 1 à 8, destiné à être utilisé dans le traitement ou la prévention de maladies ou d'affections qui peuvent être influencées par l'inhibition du cotransporteur de glucose dépendant du sodium SGLT, plus préférablement du cotransporteur de glucose dépendant du sodium SGLT2.

13. Utilisation d'un complexe cristallin entre le 1-cyano-2-(4-cyclopropyl-benzyl)-4-(ß-D-glucopyranos-1-yl)-benzène et un ou plusieurs acides aminés naturels pour la préparation d'une composition pharmaceutique,
(i) **caractérisée en ce que** l'acide aminé naturel est la L-proline, et
(ii) **caractérisée en ce que** le complexe cristallin est un complexe (1:1) entre le 1-cyano-2-(4-cyclopropyl-benzyl)-4-(ß-D-glucopyranos-1-yl)-benzène et la L-proline, et
(iii) **caractérisée en ce que** le complexe cristallin est un hydrate cristallin, et
(iv) **caractérisée en ce que** le rapport molaire du complexe cristallin de 1-cyano-2-(4-cyclopropyl-benzyl)-4-(ß-D-glucopyranos-1-yl)-benzène et de L-proline avec l'eau cristalline est de 1:1.

14. Complexe cristallin tel que défini dans la revendication 13, destiné à être utilisé dans la préparation d'une composition pharmaceutique, préférablement destiné à être utilisé dans la préparation d'une composition pharmaceutique pour le traitement ou la prévention de maladies ou d'affections qui peuvent être influencées par l'inhibition du cotransporteur de glucose dépendant du sodium SGLT, plus préférablement du cotransporteur de glucose dépendant du sodium SGLT2.
